# EUROPEAN PATENT APPLICATION

(11) **EP 4 134 873 A1**
(43) Date of publication of application: **15.02.2023**
(21) Application number: 22189754.9
(22) Date of filing: 10.08.2022
(51) Int. Cl.: G06N 3/04, G06N 3/08, G06N 5/02, G16H 10/60

(54) **NEUROSYMBOLIC DATA IMPUTATION USING AUTOENCODER AND EMBEDDINGS**

(30) Priority: 12.08.2021 US 202117401044
(71) Applicant: Accenture Global Solutions Limited, Dublin 4 (IE)
(72) Inventor: ZHENG, Xu, Dublin, D11 (IE); HAYES, Jeremiah, Dublin, D11 (IE)
(74) Representative: Swindell & Pearson Limited

(57) **Abstract**

Methods, systems and apparatus, including computer programs encoded on computer storage medium, for training a neurosymbolic data imputation system on training data inputs in a domain to impute missing data in a data input from the data domain. In one aspect a method includes, for each training data input, adding random noise to missing fields of the training data input; generating an embedding data input for the training data input using concept embeddings from the domain; processing the noisy data input and the embedding data input through a correlation network to obtain correlation data; applying attention to the noisy training data input and the correlation data to generate a combined data input; processing, by an autoencoder, the combined data input to obtain a decoded data output; computing a difference between the decoded data output and the training data input; and updating parameters of the data imputation system using the difference.

## Description

### TECHNICAL FIELD

This specification generally relates to methods, systems, and devices for data imputation.

### BACKGROUND

Data imputation is the substitution of estimated values for missing or inconsistent data fields. After substitution, a full data set can be analyzed as if the substituted values were actual observed values.

Example data imputation techniques include mean imputation, where a calculated mean of the observed values are used as a substituted value, hot deck imputation where a substituted value is randomly chosen from a data sample that has similar values on other observed values, cold deck imputation where a substituted value is systematically chosen from a data sample that has similar values on other observed values, regression imputation where a substituted value is computed by regressing the missing field on other fields, stochastic regression imputation where a substituted value is computed from a regression with a random residual value, and interpolation and extrapolation where a substituted value is estimated from other observed values in the data set.

### SUMMARY

This specification describes systems and methods for neurosymbolic data imputation using autoencoders and embeddings.

In general, one innovative aspect of the subject matter described in this specification may be embodied in methods that include the actions of for training a neurosymbolic data imputation system on multiple training data inputs in a data domain to impute missing data in a data input from the data domain, the method comprising, for each training data input: adding random noise to missing fields of the training data input to generate an updated training data input; generating an embedding data input that corresponds to the training data input using a set of concept embeddings from the data domain; processing i) the updated data input and ii) the embedding data input through a correlation network included in the neurosymbolic data imputation system to obtain correlation data representing correlations between the training data input and the embedding data input; applying an attention mechanism to the updated training data input and the correlation data to generate a combined data input; processing, by an autoencoder included in the neurosymbolic data imputation system, the combined data input to obtain a decoded data output; and computing a difference between the decoded data output and the training data input and updating values of parameters of the correlation network, encoder and decoder using the computed difference.

Other embodiments of this aspect include corresponding computer systems, apparatus, and computer programs recorded on one or more computer storage devices, each configured to perform the actions of the methods. A system of one or more computers can be configured to perform particular operations or actions by virtue of having software, firmware, hardware, or a combination thereof installed on the system that in operation causes or cause the system to perform the actions. One or more computer programs can be configured to perform particular operations or actions by virtue of including instructions that, when executed by data processing apparatus (e.g., one or more computers or computer processors), cause the apparatus to perform the actions.

The foregoing and other embodiments can each optionally include one or more of the following features, alone or in combination. In some implementations i) the data domain comprises a medical domain, ii) the set of concept embeddings comprises a set of medical concept embeddings, and iii) the training data input comprises data representing patient health information.

In some implementations i) the embedding data input comprises embeddings of diagnosis codes included in the training data input and ii) the data representing correlations between the training data input and the embedding data input comprises data representing correlations between the patient health information and the diagnosis codes included in the training data input.

In some implementations the correlation data representing correlations between the training data input and the embedding data input comprises a correlation matrix, wherein the correlation matrix comprises a number of rows equal to the size of the training data input and a number of columns equal to the size of the embedding data input.

In some implementations generating the embedding data input that corresponds to the training data input using the set of concept embeddings from the data domain comprises: identifying, from the set of concept embeddings from the data domain, concept embeddings of data included in the training data input; and including the identified concept embeddings in the embedding data input.

In some implementations applying an attention mechanism to the updated training data input and the correlation data comprises: concatenating the updated training data input and the correlation data; or multiplying the correlation data on corresponding concept values in the updated training data input.

In some implementations processing, by the autoencoder included in the neurosymbolic data imputation system, the combined data input to obtain a decoded data output comprises: processing, by an encoder included in the autoencoder, the combined data input to obtain encoded data output; processing, by a decoder included in the autoencoder, the encoded data output to obtain a decoded data output, wherein a form of the decoded data output matches a form of the training data input.

In some implementations the encoder is configured to map encoder data inputs into respective encoder data outputs in a latent space; and the decoder is configured to map decoder data inputs comprising the encoder data outputs in the latent space to respective reconstructions of the encoder data inputs.

In some implementations the latent space comprises a dimension smaller than the dimension of the training data input.

In some implementations embedding data input comprises a matrix, the matrix comprising i) a number of rows equal to the number of concept embeddings, each row corresponding to a respective concept embedding, and ii) a number of columns based on a knowledge graph or embedding model associated with the embedding data input.

In some implementations computing a difference between the decoded data output and the training data input comprises computing the difference as a loss and updating parameters of the correlation network, encoder and decoder using the computed difference comprises backpropagating loss gradients from the decoder, encoder and correlation network to determine updated values of the parameters of the correlation network, encoder and decoder.

In some implementations the method further comprises determining whether the computed difference between the decoded data output and the training data input meets a predetermined loss threshold; and in response to determining that the computed difference meets the predetermined loss threshold, terminating the training of the neurosymbolic data imputation system.

In some implementations the method further comprises determining whether the computed difference between the decoded data output has converged to within a predetermined convergence threshold; and in response to determining that the computed difference has converged to within the predetermined convergence threshold, terminating the training of the neurosymbolic data imputation system.

In some implementations the random noise is sampled from a uniform random distribution and takes values from the range (0, 0.01).

In some implementations the correlation network comprises a neural network configured, through training, to process a first data input and a second data input to generate an output that represents correlations between the first data input and the second data input.

In some implementations the method further comprises receiving a new data input from the data domain; and processing the new data input using the trained neurosymbolic data imputation system, comprising: processing the new data input using the encoder to obtain a corresponding encoder output, processing the encoder output using the decoder to obtain a corresponding decoder output, and replacing missing fields of the new data input with corresponding fields of the decoder output.

Some implementations of the subject matter described herein may realize, in certain instances, one or more of the following advantages.

A neurosymbolic data imputation system, as described in this specification, leverages additional data related to the data domain to improve data imputation. For example, in cases where the neurosymbolic data imputation system imputes data from a medical domain, the neurosymbolic data imputation system can leverage medical concept information to accurately estimate missing codes (e.g., diagnosis codes) in a patient's health information based on their existing conditions.

The details of one or more embodiments of the subject matter described in this specification are set forth in the accompanying drawings and the description below. Other potential features, aspects, and advantages of the subject matter will become apparent from the description, the drawings, and the claims.

### DESCRIPTION OF DRAWINGS

FIG. 1 is a block diagram of an example neurosymbolic data imputation system during training.
FIG. 2 is a block diagram of an example neurosymbolic data imputation system during data imputation.
FIG. 3 is a block diagram of an example neurosymbolic data imputation system.
FIG. 4 is a flow chart of an example process for training a neurosymbolic data imputation system on multiple training data inputs in a data domain to impute missing data in a data input from the data domain.
FIG. 5 is a flowchart of an example process for imputing missing data in a data input using a trained neurosymbolic data imputation system.
FIG. 6 is a schematic diagram of an exemplary computer system.

Like reference symbols in the various drawings indicate like elements.

### DETAILED DESCRIPTION

This specification describes systems and methods for neurosymbolic data imputation using autoencoders and embeddings. During training, correlations between training data inputs and concept embeddings are computed and processed with the training data inputs to train an autoencoder to impute new data inputs with improved accuracy. During runtime, the trained autoencoder can process new data inputs to determine values that can be added to missing fields of the new data inputs to produce accurate, completed data sets.

For convenience, the techniques presented in this specification are mainly described in the context of imputation of data inputs from a medical domain (e.g., estimating missing diagnosis codes in a patient health record based on their existing or historical conditions and treatments). However, the presently described techniques can also be applied to other data domains, e.g., any appropriate data domain for which corresponding concept embeddings exist. For example, job skills embeddings can be incorporated into an imputation process for HR datasets. A person could have skills in Java, Python and different web frameworks. Instead of using one-hot encodings, skill embeddings can be used to help the imputation on position or salary information in HR datasets. As another example, genome embeddings, e.g., Gene2vec, can be incorporated into an imputation process for healthcare datasets.

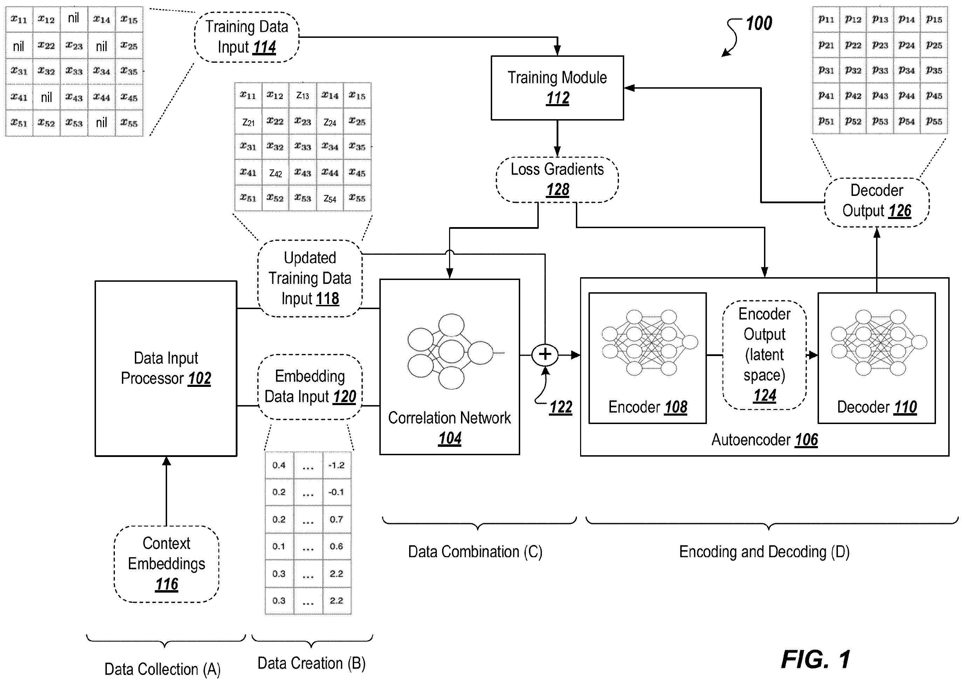

FIG. 1 is a block diagram 100 of an example neurosymbolic data imputation system during an example training process. The block diagram 100 illustrates the example training process as including four stages - a data collection stage (A), a data creation stage (B), a data combination stage (C), and an encoding and decoding stage (D). However, in some implementations the example training process may include fewer or more stages. Each of the four stages are illustrated as being performed by respective modules of the neurosymbolic data imputation system, for example, a data input processor 102, correlation network 104, autoencoder 106, and training module 112. However, in some implementations, different stages of the example training process can be performed by other computing modules.

During the data collection state (A), the data input processor 102 receives training data inputs, for example, training data input 114. In the example context, each training data input can include medical data that contains respective patient health information (e.g., a patient's age, gender, medical history, prescribed medications and diagnoses). The training data input includes one or more missing fields. For example, training data input 114 is missing the values {*x*₁₃, *x*₂₁, *x*₂₄*, x*₄₂, *x*₅₄}.

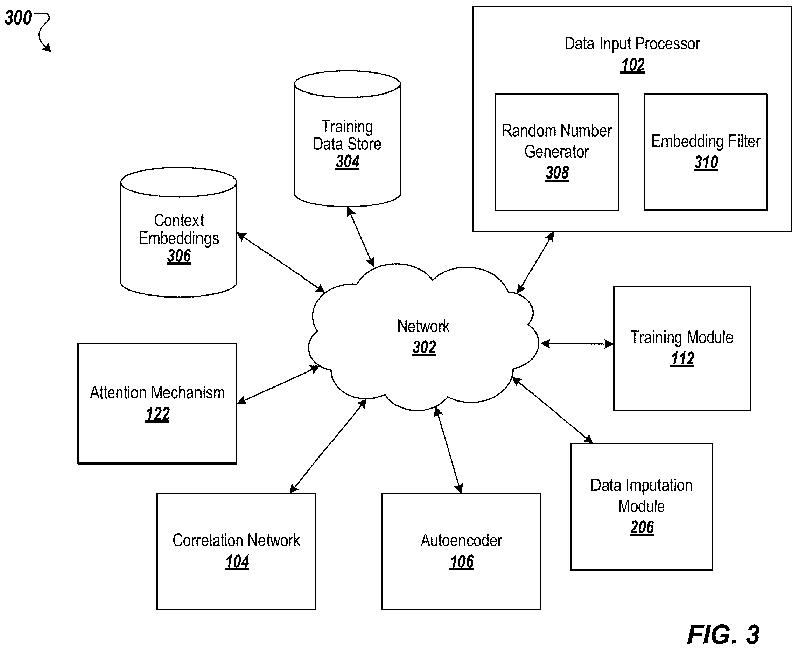

The data input processor 102 can also receive additional related information from the medical domain, for example, context embeddings 116. The context embeddings 116 can be obtained from external sources or created by the system in advance of the training process. For example, in some implementations the system can obtain embeddings for a systematically organized computer process-able collection of medical terms providing codes, terms, synonyms and definitions used in clinical documentation and reporting using a pre-trained embedding model, as described in more detail below with reference to FIG. 3. The context embeddings 116 are used to improve the data imputation of training data input 114.

During the data creation state (B), the data input processor 102 adds random noise to the missing fields of the training data input to generate an updated training data input, for example, updated training data input 118. For example, in some implementations the data input processor 102 can be configured to sample noise values from a uniform random distribution (e.g., from the range (0, 0.01)), and add the sampled noise values to respective missing fields of the training data input. For example, the missing values {*x*₁₃, *x*₂₁, *x*₂₄*, x*₄₂, *x*₅₄} in training data input 114 can be populated with randomly sampled noise values {*z*₁₃, *z*₂₁, *z*₂₄, *z*₄₂, *z*₅₄}, respectively.

The data input processor 102 also filters the context embeddings 116 using the training data input 114. For example, the data input processor 102 can filter the context embeddings 116 by selecting embeddings of data included in the training data input 114, for example, selecting embeddings of diagnosis codes included in the training data input 114. The data input processor 102 can form an embedding data input using the selected embeddings, for example, embedding data input 120.

The correlation network 104 receives the updated training data input 118 and the embedding data input 120 from the data input processor 102 and processes the updated training data input 118 and the embedding data input 120 as an input to generate an output representing correlations between the training data input 118 and the embeddings data input 120 (e.g., correlations between the patient's health information and diagnosis codes included in the embeddings data input 120). An attention mechanism 122 is applied to the output of the correlation network 104 and the updated training data input 118. For example, the system can concatenate the output of the correlation network 104 and the updated training data input 118 or can multiple the correlations on their corresponding concept values in the updated training data input 118.

During the encoding and decoding stage (D) the autoencoder 106 receives a combined data input obtained after the attention mechanism 122 is applied to the output of the correlation network 104 and the updated training data input 118. The autoencoder 106 processes the combined data input using an encoder to encode the combined data in a latent space and generate an encoder output, for example, encoder output 124. This process compresses the data from the input layer into a shorter code. The decoder 110 processes the encoded data, for example, the encoder output 124, to generate a decoded output, for example, decoder output 126. This process un-compresses the code into a format that matches the training data input 114. For example, *p*₁₁ corresponds to *x*₁₁, *p*₁₂ corresponds to *x*₁₂, etc. By construction, the decoder output 126 does not include any missing fields.

The training module 112 receives the decoder output 126 and computes a difference between the training data input 114 and the decoder output 126. The computed difference is used as a loss to train the correlation network 104, the encoder 108, and the decoder 110. For example, the training module 112 can compute loss gradients 128 and backpropagate the loss gradients 128 though the decoder 110, the encoder 108, and the correlation network 104 to determine updated values of parameters of the correlation network 104, the encoder 108, and the decoder 110.

The stages (A)-(D) can be iterated repeatedly over multiple epochs until trained values of the parameters of the correlation network 104, the encoder 108, and the decoder 110 are determined (e.g., until a loss threshold is met or until a computed loss converges).

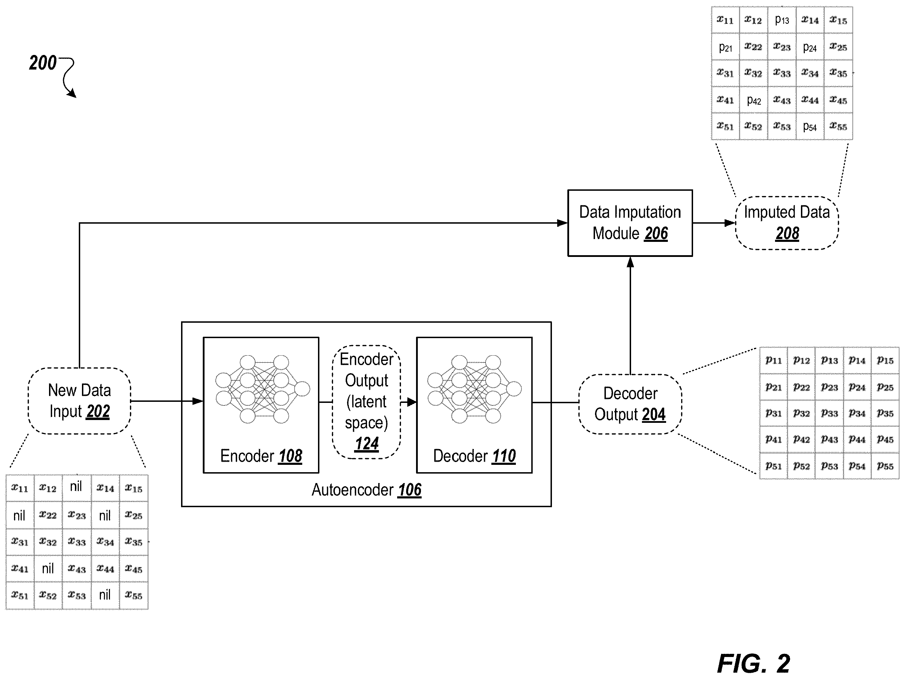

FIG. 2 is a block diagram 200 of the example neurosymbolic data imputation system shown in FIG. 1 during data imputation. After training, the autoencoder 106 receives a new data input, for example, new data input 202. The new data input 202 can include one or more missing fields. For example, the new data input 202 could include the below patient health information for a respective patient:
- Female
- age 42
- Ethnicity: Non-Hispanic

With diagnosis codes:
- Dyspnea
- Wheezing
- Hypoxemia
- Respiratory distress
where patient health information for the diagnosis codes "pneumonia" or "obesity" are missing (e.g., the patient health information does not indicate whether the patient has previously suffered from pneumonia or obesity).

The autoencoder 106 can process the new data input 202 to obtain a respective decoder output 204. A data imputation module 206 receives the decoder output 204 and the new data input 202. The data imputation module 206 identifies missing fields in the new data input 202 and populates these missing fields with corresponding values taken from the decoder output 204. For example, in FIG. 2 the new data input 202 is missing values {*x*₁₃, *x*₂₁*, x*₂₄*, x*₄₂, *x*₅₄}. The data imputation module 206 can identify the corresponding values {*p*₁₃, *p*₂₁, *p*₂₄, *p*₄₂, *p*₅₄} in the decoder output 204 and impute these values into the new data input 202 to generate an imputed data set 208, for example, a complete data set {*x*₁₁, *x*₁₂, *p*₁₃*, x*₁₄, *x*₁₅*_{,},* ... }. For example, continuing the example above, the imputed data set can include the below patient health information:
- Female
- age 42
- Ethnicity: Non-Hispanic

With diagnosis codes:
- Dyspnea
- Wheezing
- Hypoxemia
- Respiratory distress
- Pneumonia (82%, the possibility of being positive)
- Obesity (42%)
In the above imputed data set the percentages represent probabilities obtained through application of a sigmoid function in the decoder for each of the values to be imputed, where the output of the decoder represents the probabilities.

FIG. 3 is a block diagram of an example neurosymbolic data imputation system 300. The example neurosymbolic data imputation system 300 can be trained to impute missing data in a data input. The example neurosymbolic data imputation system 300 includes a network 302 (e.g., a local area network (LAN), wide area network (WLAN), the Internet, or a combination thereof). The network 302 can be accessed over a wired and/or a wireless communications link. The network 302 connects a training data store 304, context embeddings store 306, data input processor 102, correlation network 104, autoencoder 106, attention mechanism 122, training module 112, and data imputation module 206.

The training data store 304 stores multiple training data inputs from a data domain. For example, in cases where the domain is a medical domain, the training store 304 can store multiple medical records for respective patients, where each record includes medical data specifying a patient's age, gender, ethnicity, medical history, prescribed medications, and diagnoses.

The context embeddings store 306 stores embeddings of a concept corresponding to the data domain. For example, in cases where the domain is a medical domain, the context embeddings store 306 can store embeddings of medical concepts. The embeddings stored in the embeddings store 306 can be obtained from an external source or can be created by the system 300. In either case, the embeddings stored in the embeddings store 306 can obtained through a pre-trained embedding model. For example, in cases where the domain is a medical domain, the embeddings can include embeddings of a pre-defined collection of data representing medical concepts, for example, SNOMED-CT. SNOMED-CT is a collection of strings representing the medical concepts. SNOMED-CT can be formed as a graph where each vertex is a unique medical concept and related concepts are connected through labelled edges. SNOMED-CT contains medical concepts indexed by a unique concept ID (e.g., 42539006). Each medical concept is associated with a concept description, e.g., Parkinson disease. All SNOMED-CT medical concepts can be encoded, for example, as a Headache, SCTID: 25064002, into fixed size embeddings (vector representations), such that similar concepts are closer in the embedding space. The system can obtain or generate embeddings for each SNOMED-CT concept using a pre-trained embedding model.

The data input processor 102 is configured to process training data inputs, for example, obtained from the training data store 304. The data input processor 102 includes a random number generator 308 (e.g., a pseudorandom number generator) and an embedding filter 310. The random number generator 308 is configured to generate random values from a probability distribution and a given range (e.g., the range (0, 0.01)). The data input processor 102 can add random values generated by the random number generator to missing fields of training data inputs to generate complete training data inputs (also referred to herein as updated training data inputs). The embedding filter 310 is configured to identify concepts in a training data input (e.g., diagnosis codes), and construct a respective embedding matrix that includes embeddings from the context embeddings store 306 that correspond to the identified concepts.

The correlation network 104 is configured to determine correlations between two data inputs. For example, in some implementations the correlation network 104 can be a neural network that can be configured, through training, to process two data inputs, for example, an updated training data input and embedding matrix obtained from the data input processor 102, and generate an output representing correlations between the two data inputs. In cases where the data domain is a medical domain, the output of the correlation network 104 can represent correlations between a patient's patient information and diagnosis codes represented by the embedding matrix. The correlation network 104 can include parameters which can be adjusted to trained values to improve the accuracy of outputs generated by the correlation network 104.

The attention mechanism 122 is configured to combine two data inputs, for example, correlation data output by the correlation network 104 and an updated training data input generated by the data input processor 102. The attention mechanism 122 can combine data inputs by concatenating the two data inputs or by multiplying correlations on corresponding concept values in the updated training data input.

The autoencoder 106 includes an encoder that is configured to map input data into a latent space (a code), which is a distributed representation that captures its main components and the relationships of these components in the data. The autoencoder 106 also includes a decoder that is configured to map the data in the latent space (the code) to a reconstruction of the autoencoder (and encoder) input. For example, the autoencoder 106 can be a feedforward, nonrecurrent neural network. The autoencoder 106 can include parameters which can be adjusted to trained values to improve the accuracy of reconstructions generated by the autoencoder 106.

The training module 112 is configured to implement algorithms for training the correlation network 104 and autoencoder 106. For example, the training module 112 can implement backpropagation algorithms by computing a gradient of a loss function with respect to the parameters (weights) of the correlation network 104 and autoencoder 106. During training, the training module can compute differences between training data inputs obtained by the data input processor 102 and corresponding reconstructions output by the autoencoder 106 and use the computed differences as a loss to train the correlation network 104 and autoencoder 106. The training module 112 can also be configured to determine whether training termination criteria are satisfied or not and terminate a training process if the criteria are met.

The data imputation module 206 is configured to impute data inputs received during runtime, for example, after the correlation network 104 and autoencoder 106 have been trained by the training module 112. The data imputation module 206 can obtain a reconstruction of a new data input from the autoencoder 106 and use the obtained reconstruction to substitute missing values of the new data input with corresponding values in the obtained reconstruction. The data imputation module 206 can then output or store a corresponding complete data set.

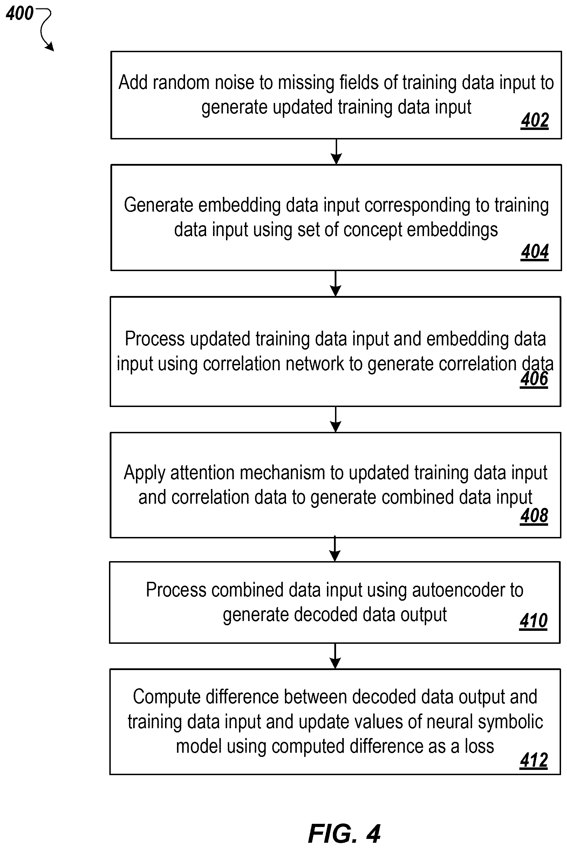

FIG. 4 is a flowchart of an example process 400 for training a neurosymbolic data imputation system on a training data input from a set of multiple training data inputs in a data domain to impute missing data in a data input from the data domain. In some implementations the data domain can be a medical domain, and the training data inputs can be data inputs representing respective patient health information. For convenience, the process 400 will be described as being performed by a system of one or more computers located in one or more locations. For example, a computing system (e.g., the computing system 300 of FIG. 3), appropriately programmed, can perform example process 400. Although the flowchart depicts the various stages of the process 400 occurring in a particular order, certain stages may in some implementations be performed in parallel or in a different order than what is depicted in the example process 400 of FIG. 4.

The system adds random noise to missing fields of the training data input to generate an updated training data input (step 402). For example, the system can identify missing fields of the training data input and, for each missing field, randomly sample a value from a predetermined range of values (e.g., randomly sample from a uniform random distribution in the range (0, 0.01)). The system can then populate the missing field with the randomly sampled value.

The system generates an embedding data input that corresponds to the training data input using a set of concept embeddings from the data domain (step 404). As described above, in some implementations the data domain can be a medical domain. In some implementations, the set of concept embeddings can be a set of medical concept embeddings (e.g., SNOMED-CT), and the embedding data input can include embeddings of diagnosis codes included in the training data input.

To generate the embedding data input the system can identify, from the set of concept embeddings from the data domain, concept embeddings of data included in the training data input and include the identified concept embeddings in the embedding data input. In other words, the system filters the set of concept embeddings by selecting embeddings of data included in the training data input and discarding embeddings of data that is not included in the training data input. For example, if the training data input includes data representing 100 different diseases, the system would generate an embedding data input that includes 100 embeddings, each embedding corresponding to a respective disease. In some implementations, the embedding data input is a matrix that includes a number of rows equal to the number of concept embeddings, each row corresponding to a respective concept embedding, and a number of columns based on the knowledge graph or embedding model associated with the embedding data input.

The system processes i) the updated data input and ii) the embedding data input through a correlation network included in the neurosymbolic data imputation system to obtain correlation data representing correlations between the training data input and the embedding data input (step 406). The correlation network can be a neural network that has been configured, through training, to process a first data input and a second data input to generate an output that represents correlations between the first data input and the second data input. The correlation data can take the form of a correlation matrix, where the correlation matrix includes a number of rows equal to the size of the training data input and a number of columns equal to the size of the embedding data input.

As described above, in some implementations the data domain can be a medical domain. In these implementations the correlation data can include data representing correlations between the patient health information and the diagnosis codes included in the training data input.

The system applies an attention mechanism to the updated training data input and the correlation data to generate a combined data input (step 408). In some implementations, the system can apply the attention mechanism by concatenating the updated training data input and the correlation data to generate a combined data input. In some implementations, the system can multiply the correlation data on corresponding concept values in the updated training data input to generate a combined data input.

The system processes the combined data input using an autoencoder included in the neurosymbolic data imputation system to obtain a decoded data output (step 410). The autoencoder includes an encoder and a decoder. The encoder is configured to map encoder data inputs into respective encoder data outputs in a latent space, where the latent space has a dimension that is smaller than the dimension of the training data input. The decoder is configured to map decoder data inputs (e.g., encoder data outputs in the latent space) to respective reconstructions of the encoder data inputs. To process the combined data input using the autoencoder, the system processes the combined data input using the encoder included in the autoencoder to obtain an encoded data output. The system then processes the encoded data output using the decoder included in the autoencoder to obtain a decoded data output. The form of the decoded data output matches the form of the training data input.

The system computes a difference between the decoded data output and the training data input. The system updates values of parameters of the correlation network, encoder and decoder using the computed difference (step 412). The system can use the computed difference as a loss and determine updated values of the parameters of the correlation network, encoder and decoder by backpropagating loss gradients from the decoder, encoder and correlation network.

The system can iteratively perform steps 402-412 over multiple epochs until each of the multiple training data inputs have been processed, until a loss threshold is met, or until changes between epochs show no improvement.

For example, in some implementations after step 412 the system can determine whether the computed difference between the decoded data output and the training data input meets a predetermined loss threshold and in response to determining that the computed difference meets the predetermined loss threshold, terminate the training of the neurosymbolic data imputation system. In response to determining that the computed difference does not meet the predetermined loss threshold, the system can perform another iteration and process a new training data input.

As another example, in some implementations, after step 412, the system can determine whether the computed difference between the decoded data output has converged to within a predetermined convergence threshold and in response to determining that the computed difference has converged to within the predetermined convergence threshold, terminate the training of the neurosymbolic data imputation system. In response to determining that the computed difference has not converged to within the predetermined convergence threshold, the system can perform another iteration and process a new training data input.

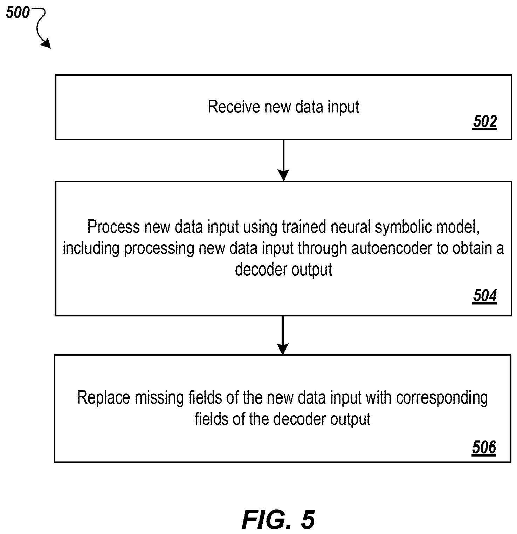

Once trained, the autoencoder included in the neurosymbolic model can be provided to impute missing fields in new data inputs, as described below with reference to FIG. 5.

FIG. 5 is a flow chart of an example process 500 for imputing missing data in a data input using a trained neurosymbolic data imputation system (e.g., a neurosymbolic data imputation system trained according to example process 400 of FIG. 4). For convenience, the process 500 will be described as being performed by a system of one or more computers located in one or more locations. For example, a computing system (e.g., the computing system 300 of FIG. 3), appropriately programmed, can perform example process 500.

The system receives a new data input (step 502). The new data input can include one or more missing fields. The new data input is a data input from the same data domain as the multiple training data inputs used to train the neurosymbolic data imputation system in example process 400 of FIG. 4.

The system processes the new data input using the trained neurosymbolic data imputation system (step 504). The system processes the new data input using the encoder included in the trained neurosymbolic data imputation system to obtain a corresponding encoder output. The system then processes the encoder output using the decoder included in the trained neurosymbolic data imputation system to obtain a corresponding decoder output.

The system replaces missing fields of the new data input with corresponding fields of the decoder output (step 506). In some implementations observed values in the new data input are not replaced or adjusted based on the decoder output. In some implementations the completed dataset can be used to determine a treatment plan for a corresponding patient. In some implementations the completed dataset can be provided as input to and processed by diagnosis systems to improve the performance of the diagnostic system. For example, the completed dataset can be used to improve the accuracy of survival prediction for lung cancer patients.

In some implementations completed medical records can be compared to original data for identification of misdiagnoses. For example, the system can compare observed values in the new data input to values generated by the decoder output. If some values differ significantly, e.g., more than a predetermined acceptable threshold, the system can output a prompt, e.g., through a user interface, that flags the differing values as requiring investigation.

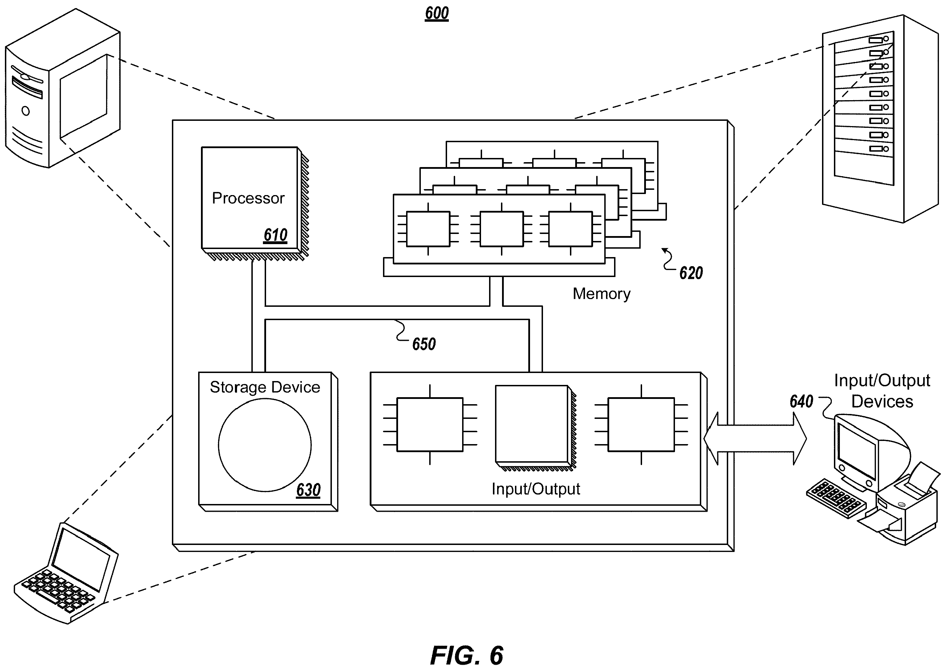

FIG. 6 is a schematic diagram of an exemplary computer system 600. The system 600 can be used for the operations described in association with the processes 400 and 500 described above according to some implementations. The system 600 is intended to represent various forms of digital computers, such as laptops, desktops, workstations, personal digital assistants, servers, blade servers, mainframes, mobile devices and other appropriate computers. The components shown here, their connections and relationships, and their functions, are exemplary only, and do not limit implementations of the inventions described and/or claimed in this document.

The system 600 includes a processor 610, a memory 620, a storage device 630, and an input/output device 640. Each of the components 610, 620, 630, and 620 are interconnected using a system bus 650. The processor 610 may be enabled for processing instructions for execution within the system 600. In one implementation, the processor 610 is a single-threaded processor. In another implementation, the processor 610 is a multi-threaded processor. The processor 610 may be enabled for processing instructions stored in the memory 620 or on the storage device 630 to display graphical information for a user interface on the input/output device 640.

The memory 620 stores information within the system 600. In one implementation, the memory 620 is a computer-readable medium. In one implementation, the memory 620 is a volatile memory unit. In another implementation, the memory 620 is a non-volatile memory unit.

The storage device 630 may be enabled for providing mass storage for the system 600. In one implementation, the storage device 630 is a computer-readable medium. In various different implementations, the storage device 630 may be a floppy disk device, a hard disk device, an optical disk device, or a tape device.

The input/output device 640 provides input/output operations for the system 600. In one implementation, the input/output device 640 includes a keyboard and/or pointing device. In another implementation, the input/output device 640 includes a display unit for displaying graphical user interfaces.

Embodiments and all of the functional operations described in this specification may be implemented in digital electronic circuitry, or in computer software, firmware, or hardware, including the structures disclosed in this specification and their structural equivalents, or in combinations of one or more of them. Embodiments may be implemented as one or more computer program products, *i.e.,* one or more modules of computer program instructions encoded on a computer readable medium for execution by, or to control the operation of, data processing apparatus. The computer readable medium may be a machine-readable storage device, a machine-readable storage substrate, a memory device, a composition of matter effecting a machine-readable propagated signal, or a combination of one or more of them. The term "data processing apparatus" encompasses all apparatus, devices, and machines for processing data, including by way of example a programmable processor, a computer, or multiple processors or computers. The apparatus may include, in addition to hardware, code that creates an execution environment for the computer program in question, e.g., code that constitutes processor firmware, a protocol stack, a database management system, an operating system, or a combination of one or more of them. A propagated signal is an artificially generated signal, *e.g.,* a machine-generated electrical, optical, or electromagnetic signal that is generated to encode information for transmission to suitable receiver apparatus.

A computer program (also known as a program, software, software application, script, or code) may be written in any form of programming language, including compiled or interpreted languages, and it may be deployed in any form, including as a stand alone program or as a module, component, subroutine, or other unit suitable for use in a computing environment. A computer program does not necessarily correspond to a file in a file system. A program may be stored in a portion of a file that holds other programs or data (*e.g.*, one or more scripts stored in a markup language document), in a single file dedicated to the program in question, or in multiple coordinated files (*e.g*., files that store one or more modules, sub programs, or portions of code). A computer program may be deployed to be executed on one computer or on multiple computers that are located at one site or distributed across multiple sites and interconnected by a communication network.

The processes and logic flows described in this specification may be performed by one or more programmable processors executing one or more computer programs to perform functions by operating on input data and generating output. The processes and logic flows may also be performed by, and apparatus may also be implemented as, special purpose logic circuitry, e.g., an FPGA (field programmable gate array) or an ASIC (application specific integrated circuit).

Processors suitable for the execution of a computer program include, by way of example, both general and special purpose microprocessors, and any one or more processors of any kind of digital computer. Generally, a processor will receive instructions and data from a read only memory or a random access memory or both.

The essential elements of a computer are a processor for performing instructions and one or more memory devices for storing instructions and data. Generally, a computer will also include, or be operatively coupled to receive data from or transfer data to, or both, one or more mass storage devices for storing data, e.g., magnetic, magneto optical disks, or optical disks. However, a computer need not have such devices. Moreover, a computer may be embedded in another device, *e.g.,* a tablet computer, a mobile telephone, a personal digital assistant (PDA), a mobile audio player, a Global Positioning System (GPS) receiver, to name just a few. Computer readable media suitable for storing computer program instructions and data include all forms of non volatile memory, media and memory devices, including by way of example semiconductor memory devices, *e.g.,* EPROM, EEPROM, and flash memory devices; magnetic disks, *e.g.,* internal hard disks or removable disks; magneto optical disks; and CD ROM and DVD-ROM disks. The processor and the memory may be supplemented by, or incorporated in, special purpose logic circuitry.

To provide for interaction with a user, embodiments may be implemented on a computer having a display device, *e.g.,* a CRT (cathode ray tube) or LCD (liquid crystal display) monitor, for displaying information to the user and a keyboard and a pointing device, *e.g.,* a mouse or a trackball, by which the user may provide input to the computer. Other kinds of devices may be used to provide for interaction with a user as well; for example, feedback provided to the user may be any form of sensory feedback, e.g., visual feedback, auditory feedback, or tactile feedback; and input from the user may be received in any form, including acoustic, speech, or tactile input.

Embodiments may be implemented in a computing system that includes a back end component, *e.g.,* as a data server, or that includes a middleware component, *e.g.,* an application server, or that includes a front end component, e.g., a client computer having a graphical user interface or a Web browser through which a user may interact with an implementation, or any combination of one or more such back end, middleware, or front end components. The components of the system may be interconnected by any form or medium of digital data communication, e.g., a communication network. Examples of communication networks include a local area network ("LAN") and a wide area network ("WAN"), e.g., the Internet.

The computing system may include clients and servers. A client and server are generally remote from each other and typically interact through a communication network. The relationship of client and server arises by virtue of computer programs running on the respective computers and having a client-server relationship to each other.

While this specification contains many specifics, these should not be construed as limitations on the scope of the disclosure or of what may be claimed, but rather as descriptions of features specific to particular embodiments. Certain features that are described in this specification in the context of separate embodiments may also be implemented in combination in a single embodiment. Conversely, various features that are described in the context of a single embodiment may also be implemented in multiple embodiments separately or in any suitable subcombination. Moreover, although features may be described above as acting in certain combinations and even initially claimed as such, one or more features from a claimed combination may in some cases be excised from the combination, and the claimed combination may be directed to a subcombination or variation of a subcombination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the embodiments described above should not be understood as requiring such separation in all embodiments, and it should be understood that the described program components and systems may generally be integrated together in a single software product or packaged into multiple software products.

In each instance where an HTML file is mentioned, other file types or formats may be substituted. For instance, an HTML file may be replaced by an XML, JSON, plain text, or other types of files. Moreover, where a table or hash table is mentioned, other data structures (such as spreadsheets, relational databases, or structured files) may be used.

Thus, particular embodiments have been described. Other embodiments are within the scope of the following claims. For example, the actions recited in the claims may be performed in a different order and still achieve desirable results.

## Claims

1. A computer implemented method for training a neurosymbolic data imputation system on multiple training data inputs in a data domain to impute missing data in a data input from the data domain, the method comprising, for each training data input:
adding random noise to missing fields of the training data input to generate an updated training data input;
generating an embedding data input that corresponds to the training data input using a set of concept embeddings from the data domain;
processing i) the updated data input and ii) the embedding data input through a correlation network included in the neurosymbolic data imputation system to obtain correlation data representing correlations between the training data input and the embedding data input;
applying an attention mechanism to the updated training data input and the correlation data to generate a combined data input;
processing, by an autoencoder included in the neurosymbolic data imputation system, the combined data input to obtain a decoded data output; and
computing a difference between the decoded data output and the training data input and updating values of parameters of the correlation network, encoder and decoder using the computed difference.

2. The method of claim 1, wherein i) the data domain comprises a medical domain, ii) the set of concept embeddings comprises a set of medical concept embeddings, and iii) the training data input comprises data representing patient health information, wherein, optionally: i) the embedding data input comprises embeddings of diagnosis codes included in the training data input and ii) the data representing correlations between the training data input and the embedding data input comprises data representing correlations between the patient health information and the diagnosis codes included in the training data input.

3. The method of claim 1 or 2, wherein the correlation data representing correlations between the training data input and the embedding data input comprises a correlation matrix, wherein the correlation matrix comprises a number of rows equal to the size of the training data input and a number of columns equal to the size of the embedding data input.

4. The method of claim 1, 2 or 3, wherein generating the embedding data input that corresponds to the training data input using the set of concept embeddings from the data domain comprises:
identifying, from the set of concept embeddings from the data domain, concept embeddings of data included in the training data input; and
including the identified concept embeddings in the embedding data input.

5. The method of any of the preceding claims, wherein applying an attention mechanism to the updated training data input and the correlation data comprises:
concatenating the updated training data input and the correlation data; or
multiplying the correlation data on corresponding concept values in the updated training data input.

6. The method of any of the preceding claims, wherein processing, by the autoencoder included in the neurosymbolic data imputation system, the combined data input to obtain a decoded data output comprises:
processing, by an encoder included in the autoencoder, the combined data input to obtain encoded data output;
processing, by a decoder included in the autoencoder, the encoded data output to obtain a decoded data output, wherein a form of the decoded data output matches a form of the training data input;
and wherein, optionally:
the encoder is configured to map encoder data inputs into respective encoder data outputs in a latent space; and
the decoder is configured to map decoder data inputs comprising the encoder data outputs in the latent space to respective reconstructions of the encoder data inputs, wherein, optionally, the latent space comprises a dimension smaller than the dimension of the training data input.

7. The method of any of the preceding claims, wherein embedding data input comprises a matrix, the matrix comprising i) a number of rows equal to the number of concept embeddings, each row corresponding to a respective concept embedding, and ii) a number of columns based on a knowledge graph or embedding model associated with the embedding data input.

8. The method of any of the preceding claims, wherein computing a difference between the decoded data output and the training data input comprises computing the difference as a loss and updating parameters of the correlation network, encoder and decoder using the computed difference comprises backpropagating loss gradients from the decoder, encoder and correlation network to determine updated values of the parameters of the correlation network, encoder and decoder.

9. The method of any of the preceding claims, further comprising:
determining whether the computed difference between the decoded data output and the training data input meets a predetermined loss threshold; and
in response to determining that the computed difference meets the predetermined loss threshold, terminating the training of the neurosymbolic data imputation system.

10. The method of any of the preceding claims, further comprising:
determining whether the computed difference between the decoded data output has converged to within a predetermined convergence threshold; and
in response to determining that the computed difference has converged to within the predetermined convergence threshold, terminating the training of the neurosymbolic data imputation system.

11. The method of any of the preceding claims, wherein the random noise is sampled from a uniform random distribution and takes values from the range (0, 0.01), and/or wherein the correlation network comprises a neural network configured, through training, to process a first data input and a second data input to generate an output that represents correlations between the first data input and the second data input.

12. The method of any of the preceding claims, further comprising:
receiving a new data input from the data domain; and
processing the new data input using the trained neurosymbolic data imputation system, comprising:
processing the new data input using the encoder to obtain a corresponding encoder output,
processing the encoder output using the decoder to obtain a corresponding decoder output, and
replacing missing fields of the new data input with corresponding fields of the decoder output.

13. A computer-readable storage medium comprising instructions stored thereon that are executable by at least one processing device and upon such execution cause the at least one processing device to perform the method of any of the preceding claims.

14. A system comprising one or more computers and one or more storage devices storing instructions that are operable, when executed by the one or more computers, to cause the one or more computers to perform operations for training a neurosymbolic data imputation system on multiple training data inputs in a data domain to impute missing data in a data input from the data domain, the operations comprising, for each training data input:
adding random noise to missing fields of the training data input to generate an updated training data input;
generating an embedding data input that corresponds to the training data input using a set of concept embeddings from the data domain;
processing i) the updated data input and ii) the embedding data input through a correlation network included in the neurosymbolic data imputation system to obtain correlation data representing correlations between the training data input and the embedding data input;
applying an attention mechanism to the updated training data input and the correlation data to generate a combined data input;
processing, by an autoencoder included in the neurosymbolic data imputation system, the combined data input to obtain a decoded data output; and
computing a difference between the decoded data output and the training data input and updating values of parameters of the correlation network, encoder and decoder using the computed difference.

15. The system of claim 13, wherein the instructions are operable, when executed by the one or more computers, to cause the one or more computers to perform operations defined in one or more of claims 1 to 12.
